# EUROPÄISCHE PATENTSCHRIFT

(11) **EP 1 353 613 B1**
(45) Veröffentlichungstag und Bekanntmachung des Hinweises auf die Patenterteilung: **26.09.2007**
(21) Anmeldenummer: 01978440.4
(22) Anmeldetag: 19.10.2001
(51) Int. Cl.: A61F 2/34

(54) **SPREIZPFANNE**
EXPANDABLE SOCKET
COTYLE EXTENSIBLE

(30) Priorität: 26.01.2001 DE 10103482
(43) Veröffentlichungstag der Anmeldung: 22.10.2003
(73) Patentinhaber: Mathys Medizinaltechnik AG, 2544 Bettlach (CH)
(72) Erfinder: BARBIERI, Enzo, 50054 Fucecchio (IT); RENZINI, Renzo, Ospedale Riuniti S. Maria, 52043 Castiglion Fiorentiono (IT); COTTING, Anton, CH-2540 Grenchen (CH); CHRISTEN, Peter, CH-25245 Selzach (CH); DELFOSSE, Daniel, CH-3018 Bern (CH)
(74) Vertreter: Körfer, Thomas
(86) Internationale Anmeldenummer: PCT/EP2001/012130
(87) Internationale Veröffentlichungsnummer: WO 2002/058598

(56) Entgegenhaltungen:
- EP-A- 0 169 978
- EP-A- 0 242 633
- DE-U- 20 001 940
- US-A- 5 226 917

## Beschreibung

Die Erfindung betrifft eine Spreizpfanne zur Ausbildung einer Gelenkverbindung zwischen zwei menschlichen oder tierischen Knochen.

Aus der EP 0 169 978 B1 ist eine aus einem inneren Pfannenkörper und einer Außenschale bestehende, für eine zementfreie Verankerung geeignete Endoprothese für eine Hüftgelenkspfanne bekannt, bei der der Pfannenkörper mit einem konischen Mantel und die Außenschale mit einem an den Mantel angepaßten Hohlraum versehen ist, wobei die Außenschale und der Pfannenkörper über eine Verriegelungsstruktur ineinander gehalten sind, wobei ferner auf dem Umfang der außen mindestens annähernd Halbkugelform aufweisenden Außenschale eine Anzahl in Meridianrichtung verlaufender Schlitze gleichmäßig verteilt sind und wobei ferner die Oberfläche der Außenschale mit einer aus Vorsprüngen bestehenden Struktur versehen ist.

Ferner ist aus der EP 0 242 633 B1 ebenfalls eine aus einem inneren Pfannenkörper und einer Außenschale bestehende, für eine zementfreie Verankerung geeignete Endoprothese für eine Hüftgelenkspfanne bekannt, welche ähnlich der aus der EP 0 169 978 B1 bekannten Endoprothese aufgebaut ist und im zum Äquator gelegenen Bereich der Außenschalenoberfläche eine aus mehreren peripheren Reihen angeordneten Vorsprüngen bestehende Struktur aufweist.

Die Vorsprünge gemäß EP 0 242 633 B1 sind dornenförmig geformt und auf konzentrischen Kreisen auf der Oberfläche der Spreizpfanne angeordnet. Die Vorsprünge gemäß EP 0 169 978 B1 sind stiftförmig ausgebildet und auf die Oberfläche der Spreizpfanne aufgesetzt bzw. in diese in entsprechende Öffnungen eingesetzt.

Nachteilig an den aus den obengenannten Druckschriften bekannten Spreizpfannen für Hüftgelenksendoprothesen ist insbesondere, daß die Vorsprünge auf der Spreizpfanne so angeordnet sind, daß Kräfte, die entgegen der Implantationsrichtung der Spreizpfanne im Knochengewebe wirken, dazu führen können, daß sich die Spreizpfanne aus dem Knochengewebe löst. Dies wird insbesondere dadurch begünstigt, daß die Vorsprünge entlang von Meridianen auf der Oberfläche der Halbkugel angeordnet sind, die sich alle - ähnlich wie Längenkreise auf einem Globus - in einem Punkt, nämlich dem Pol der Halbkugel, treffen.

Weiterhin ist die Form der Vorsprünge ungünstig gewählt, da sie beim Eindringen in das spongiose Knochengewebe einerseits zu Verdichtungen und andererseits zu Löchern führen können. Dadurch ist die Haltekraft im Knochengewebe beeinträchtigt, wodurch es ebenfalls zu einem Herauslösen der Spreizpfanne aus dem spongiosen Gewebe kommen kann.

Aufgabe der Erfindung ist es somit, eine Oberflächenstruktur zu schaffen, deren Vorsprünge beim Eindringen in den Knochen einerseits eine allseitig gleichmäßige Verdichtung des von ihnen verdrängten Gewebes und andererseits überall die Voraussetzung für ein gutes Anhaften und Anwachsen des Gewebes bietet. Ferner sollte die Struktur, die durch die Vorsprünge gebildet wird, so beschaffen sein, daß die Haltekraft im spongiosen Gewebe durch die spezielle Anordnung der Vorsprünge verstärkt wird.

Die Aufgabe wird durch die Merkmale des Anspruchs 1 gelöst.

Die Aufgabe wird erfindungsgemäß dadurch gelöst, daß die Vorsprünge so auf Lappen der Spreizpfanne angeordnet sind, daß mittig auf dem Lappen angeordnete Reihen von Vorsprüngen auf einem Meridian der halbkugeligen Spreizpfanne verlaufen, während zumindest eine benachbarte Reihe von Vorsprüngen auf einer Linie verläuft, die parallel zum Meridian der mittig angeordneten Reihen von Vorsprüngen und nicht durch den Pol der halbkugeligen Spreizpfanne verlaufen.

Die Ansprüche 2 bis 12 beinhalten vorteilhafte Weiterbildungen der Erfindung.

Im bevorzugten Ausführungsbeispiel beträgt die Anzahl von Reihen von Vorsprüngen auf den Lappen, die durch meridial verlaufende Schlitze voneinander getrennt sind, drei.

Bevorzugt weisen die Vorsprünge in den drei Reihen auf jedem der Lappen gleiche Abstände auf.

Vorteilhafterweise erheben sich die Vorsprünge mit zunehmender Entfernung von einem Äquator der Spreizpfanne abnehmend hoch über der Oberfläche der Spreizpfanne. Die Höhe der Vorsprünge ist mit Hinblick auf die Geometrie der zusammengepreßten Spreizpfanne beim Einsetzen in den Knochen so optimiert, daß ein Anstoßen und Einschneiden der Vorsprünge am Knochen verhindert wird.

Die Vorsprünge der mittigen Reihe sind symmetrisch geformt, während die Vorsprünge der benachbarten Reihen asymmetrisch geformt sind. Insbesondere sind die Flanken der Vorsprünge der mittigen Reihe senkrecht zur Oberfläche der Spreizpfanne, während die Flanken der Vorsprünge der benachbarten Reihen mit der Oberfläche der Spreizpfanne einen Winkel einschließen, der größer als 90° ist.

Zur einfacheren Implantation weist die Spreizpfanne an ihrem Äquator eine nach radial innen gerichtete Abschrägung der Lappen auf. Im Bereich der äquatorialen Abschrägung ist vorteilhafterweise in jedem Lappen eine Nut vorgesehen. Die Nut dient als Stabilisierung für den in die Spreizpfanne eingelegten Gleitkörper. Im bevorzugten Ausführungsbeispiel gestatten die Anordnung der Nuten, einen nichtrotationssymmetrischen Gleitkörper interoperativ an die Gegebenheiten des Operationsfeldes anzupassen, wobei der Gleitkörper jeweils um den Winkel von 30° rotiert und eingesetzt werden kann.

Die Erfindung wird nachfolgend anhand eines bevorzugten Ausführungsbeispiels unter Bezugnahme auf die Zeichnung näher beschrieben. In der Zeichnung zeigen:
- Fig. 1: eine schematische Gesamtdarstellung eines Ausführungsbeispiels einer erfindungsgemäßen Spreizpfanne,
- Fig. 2: eine schematische Aufsicht vom Äquator in Richtung auf den Pol der erfindungsgemäßen Spreizpfanne gemäß Fig. 1,
- Fig. 3: einen schematischen Längsschnitt durch die erfindungsgemäße Spreizpfanne entlang der Linie III-III in Fig. 2, und
- Fig. 4: eine perspektivische Teildarstellung der erfindungsgemäß ausgestalteten Spreizpfanne im Bereich IV in Fig. 2.

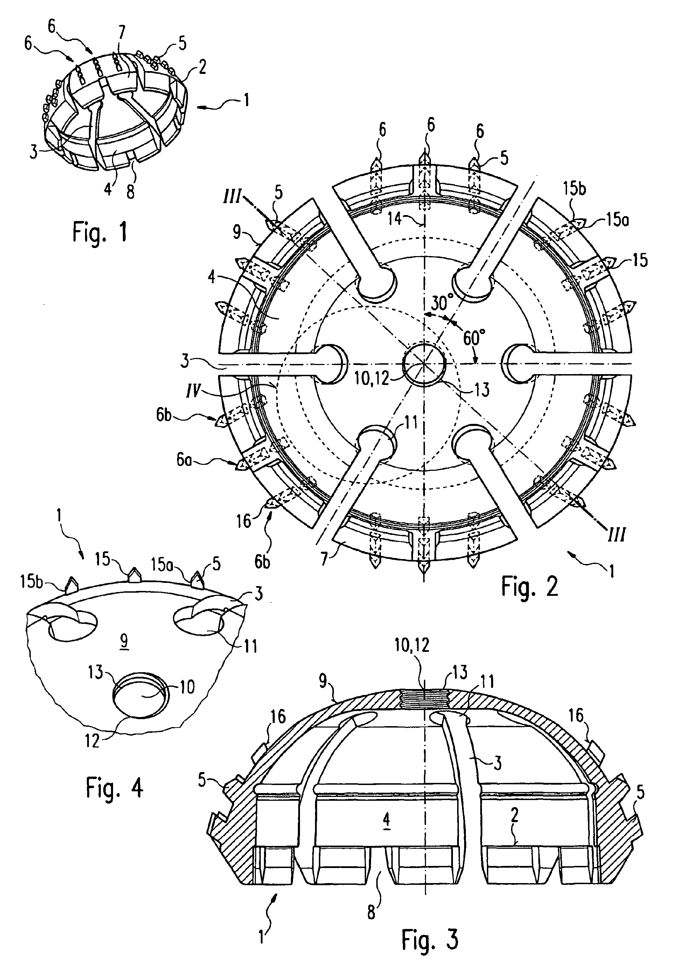

Fig. 1 zeigt in einer schematischen Gesamtdarstellung ein Ausführungsbeispiel einer erfindungsgemäßen Spreizpfanne 1. Die Spreizpfanne 1 ist dabei insbesondere als Teil einer Endoprothese, beispielsweise einer Hüftprothese, zu verwenden, wobei ein auf die Gelenkkugel des Hüftgelenkes aufgesetzte Gleitkörper, welche beispielsweise aus Kunststoff bestehen kann, in die Spreizpfanne 1 eingesetzt wird.

Die Spreizpfanne 1 ist in Fig. 1 in einer perspektivischen Darstellung mit Blick von seitlich unten dargestellt. Sie weist mehrere, in meridialer Richtung von einem Äquator 2 der zumindest annähernd halbkugelförmig ausgebildeten Spreizpfanne 1 ausgehende Schlitze 3 auf, welche sich vom Äquator 2 der Spreizpfanne 1 in Richtung auf den Pol, welcher in Fig. 1 nicht sichtbar ist, erstrecken. Die Schlitze 3 teilen zumindest den äquatornahen Teil der Spreizpfanne 1 in Lappen 4 ein, deren Anzahl im vorliegenden Ausführungsbeispiel gleich der Anzahl der meridialen Schlitze 3 ist. Durch die Schlitze 3 erhält die Spreizpfanne 1 zumindest im äquatorialen Bereich eine gewisse Elastizität, welche mit Hilfe eines geeigneten, nicht weiter dargestellten Implantationsinstruments die Implantation der Spreizpfanne 1 in entsprechend vorbereitetes Knochengewebe des Beckens ermöglicht.

An der Außenseite der Lappen 4 sind Vorsprünge 5 in Reihen 6 angeordnet. Im vorliegenden Ausführungsbeispiel sind auf jedem Lappen 4 drei Reihen 6 von Vorsprüngen 5 ausgebildet. Die Vorsprünge 5 sind dabei so angeordnet und geformt, daß sie eine zementfreie Verankerung der Spreizpfanne 1 vorwiegend im spongiosen Gewebe des Beckens ermöglichen.

Am Äquator 2 der Spreizpfanne 1 weisen die Lappen 4 eine Abschrägung 7 auf, welche nach radial innen geneigt ist. Weiterhin weisen die Lappen 4 im Bereich der Abschrägung 7 jeweils zumindest eine Nut 8 auf.

Fig. 2 zeigt eine schematische Aufsicht auf die erfindungsgemäße ausgestaltete Spreizpfanne 1 mit Blickrichtung vom Äquator 2 in Richtung des Pols 10. Die erfindungsgemäße Spreizpfanne 1 weist im vorliegenden bevorzugten Ausführungsbeispiel sechs meridiale Schlitze 3 und eben so viele dazwischenliegende, durch die Schlitze 3 voneinander getrennte Lappen 4 auf. Jeder der Lappen 4 umfaßt, wie bereits in Fig. 1 angedeutet, drei Reihen 6 von Vorsprüngen 5, die auf einer äußeren Oberfläche 9 der Spreizpfanne 1 angeordnet sind.

Die meridialen Schlitze 3 weisen an ihren polseitigen Enden kreisförmige Erweiterungen 11 auf, die den bei der Implantation durch das Instrument wirkenden Kräften Rechnung tragen.

Am Pol 10 der Spreizpfanne 1 ist eine Bohrung 12 vorgesehen, die beispielsweise über ein Gewinde 13 verfügen kann. Dieses kann beispielsweise zur Fixierung der Spreizpfanne 1 am Implantationsinstrument dienen.

Besonderes Augenmerk ist auf die spezielle Form und Anordnung der Vorsprünge 5 auf der Oberfläche 9 der Spreizpfanne 1 zu richten. Zum besseren Verständnis der erfindungsgemäßen Anordnung der Vorsprünge 5 auf der Spreizpfanne 1 ist es dienlich, die Figuren 1 und 2 gemeinsam zu betrachten. Auf dem in Fig. 1 dem Betrachter zugewandten Lappen 4 der Spreizpfanne 1 sind drei Reihen 6 von Vorsprüngen 5 zu erkennen. Eine mittige Reihe 6a wird dabei von zwei benachbarten Reihen 6b flankiert. Die mittige Reihe 6a ist dabei auf einem Meridian 14 der halbkugelförmigen Spreizpfanne 1 angeordnet. Der Meridian 14 läuft dabei vergleichbar den Längenkreisen auf einem Globus durch den Pol 10 der Spreizpfanne 1. Die benachbarten Reihen 6b von Vorsprüngen 5 sind auf Parallelen zu dem Meridian 14 angeordnet, welche in jedem Punkt parallel zu dem Meridian 14 verlaufen und damit den Pol 10 der Spreizpfanne 1 nicht schneiden. Alle Reihen 6 von Vorsprüngen 5 stehen senkrecht zum Äquator 2 der Spreizpfanne 1.

Im vorliegenden Ausführungsbeispiel sind sechs meridiale Schlitze 3, sechs Lappen 4 und sechs Meridiane 14, auf welchen die mittige Reihe 6a von Vorsprüngen angeordnet ist, vorhanden. Die meridialen Schlitze 3 sind jeweils in einem Winkelabstand von 60° angebracht, so daß auch die Meridiane 14 der sechs mittigen Reihen 6a einen Winkel von 60° einschließen. Somit sind die Meridiane 14 jeweils unter einem Winkel von 30° gegenüber den meridialen Schlitzen 3 versetzt.

Dadurch, daß die Vorsprünge 5 auf drei zueinander parallelen Reihen 6 angeordnet sind, verringert sich der Abstand der Vorsprünge 5 von den meridialen Schlitzen 3 mit zunehmender Entfernung vom Äquator 2 der Spreizpfanne 1. Dies wird in Fig. 2 aus den gestrichelt angedeuteten Vorsprüngen 5, die in der Darstellung in Fig. 2 hinter der Krümmung der halbkugeligen Spreizpfanne 1 verborgen sind, verdeutlicht. Im vorliegenden Ausführungsbeispiel sind über den äquatorialen Vorsprüngen 5 jeweils zwei weitere Vorsprünge 5 angeordnet. Pro Lappen 4 sind damit neun Vorsprünge 5 vorgesehen. Die Vorsprünge 5 sind vom Äquator 2 der Spreizpfanne 1 in Richtung Pol 10 der Spreizpfanne 1 auf konzentrischen Kreisen, die sich vom Äquator 2 zum Pol 10 verjüngen, angeordnet.

Die Form der Vorsprünge 5 ist im Querschnitt rechteckig mit einem aufgesetztem, dachförmigen Dreieck, was in räumlicher Form einem Quader mit einem aufgesetztem dreikantigem Prisma entspricht. Die Vorsprünge 5 der mittigen Reihe 6a sind dabei symmetrisch geformt, d. h., daß Flanken 15 der Vorsprünge 5 in der mittigen Reihe 6a gleich lang sind und mit der Oberfläche 9 der Spreizpfanne 1 einen Winkel von 90° einschließen. Die Vorsprünge 5 der benachbarten Reihen 6b sind achsenparallel zu den Vorsprüngen 5 der mittigen Reihe 6a ausgebildet. Das bedeutet, daß der mittigen Reihe 6a zugewandte Flanken 15a der Vorsprünge 5 der benachbarten Reihen 6b kürzer sind als der mittigen Reihe 6a abgewandte Flanken 15b und die Flanken 15b mit der Oberfläche 9 der Spreizpfanne 1 einen Winkel einschließen, der größer als 90° ist. Entsprechend schließen die Flanken 15a mit der Oberfläche 9 der Spreizpfanne 1 einen Winkel ein, der kleiner als 90° ist. Somit sind sowohl die Reihen 6, auf denen die Vorsprünge 5 angeordnet sind, als auch die Achsen der Vorsprünge 5 auf jedem Lappen 4 parallel zueinander ausgerichtet.

In der in Fig. 2 dargestellten Aufsicht vom Äquator 2 zum Pol 10 der Spreizpfanne 1 sind Giebelseiten 16 der Vorsprünge 5 erkennbar. Die Neigung der Giebelseiten 16 der Vorsprünge 5 ist dabei so gewählt, daß die Vorsprünge 5 leicht gegenüber einer Implantationsrichtung der Spreizpfanne 1 geneigt sind. Im gespreizten Zustand wird dadurch ein leicht hinterschnittenes Volumen in Richtung der Implantation gebildet. Wird die Spreizpfanne 1 in das nicht dargestellte Implantationsinstrument eingespannt, sind die Vorsprünge nicht mehr entgegen der Implantationsrichtung geneigt. Dadurch kann erreicht werden, daß bei der Implantation das Gewebe im Bereich der Vorsprünge 5 weder stark verdichtet wird, noch nach der Implantation bzw. dem Einspreizen der Spreizpfanne 1 im Gewebe Hohlräume hinterläßt, die ein schlechteres Einwachsen des Knochens bedingen könnten. Die Drehachse beim Öffnen der zum Einsetzen zusammengepreßten Spreizpfanne 1 liegt für jeden Lappen 4 auf der Verbindung der beiden benachbarten kreisförmigen Erweiterungen 11.

Der Vorteil der auf parallelen Reihen 6a und 6b angeordneten Vorsprünge 5 wird deutlich, wenn die Folgen einer Zugkraft entgegen der Implantationsrichtung der Spreizpfanne 1 betrachtet werden. Diese Zugkraft wird beispielsweise durch das Eigengewicht des betroffenen Beines auf das Implantat ausgeübt.

Wären die Reihen 6a und 6b von Vorsprüngen 5, wie aus dem Stand der Technik bekannt, alle auf Meridianen 14 angeordnet, wäre die Gefahr des Herausrutschens der Spreizpfanne 1 aus dem spongiosen Knochengewebe bedingt durch die hintereinanderliegenden Vorsprünge 5 vergrößert. Die auf parallelen Reihen 6 angeordneten Vorsprünge 5 sind in Bezug auf eine Zugrichtung jedoch nicht hintereinander, sondern seitlich versetzt angeordnet. Damit wird der Widerstand gegen eine etwaige Zugbewegung im Bereich der benachbarten Reihen 6b von einem Vorsprung 5 am Äquator der Spreizpfanne 1 bei auf Meridianen angeordneten Vorsprüngen 5 zusätzlich auf alle drei Vorsprünge 5 der jeweils rechts und links benachbarten Reihen 6b ausgedehnt, so daß die resultierende Anzahl von Vorsprüngen 5, die die Spreizpfanne 1 pro Lappen 4 entgegen einer Zugkraft im Gewebe halten, von drei auf sieben ansteigt. Dabei hat die achsenparallele Anordnung der einzelnen Vorsprünge 5 in den benachbarten Reihen 6b relativ zur mittigen Reihe 6a eine weitere unterstützende Wirkung. Die Haltbarkeit der Spreizpfanne 1 im spongiosen Knochengewebe der Hüfte wird dadurch erheblich verstärkt.

Fig. 3 zeigt eine Schnittdarstellung durch die erfindungsgemäße Spreizpfanne 1 entlang einer Linie III-III in Fig. 2. Dabei ist der Hintergrund der geschnittenen Halbkugel der Spreizpfanne 1 zur Verdeutlichung mitdargestellt.

Anhand von Fig. 3 kann die vorher angesprochene, leicht hinterschnittene Form der Vorsprünge 5 verdeutlicht werden. Die Hinterschneidung ist dabei so gewählt, daß die Vorsprünge 5 im entspannten Zustand der Spreizpfanne 1 geringfügig in Implantationsrichtung hinterschnitten sind, was im gespannten Zustand, etwa wenn die Spreizpfanne 1 in das Implantationsinstrument eingespannt ist, aufgehoben ist. Bei der Implantation stehen die Vorsprünge 5 in Implantationsrichtung, treten also gerade in das Knochengewebe der Hüfte ein. Wird danach das Implanationsinstrument entfernt, spreizt sich die Spreizpfanne 1 in das spongiose Gewebe ein und treibt dabei die Vorsprünge 5 unter einem Winkel, welcher etwas über 90° liegt, ein. Die Verdichtung des Gewebes bzw. die Bildung von Hohlräumen durch zu starke Hinterschneidung, was zu unvollständiger Umwachsung durch das spongiose Gewebe führen kann, wird dadurch vermieden.

Ebenso ist in Fig. 3 zu erkennen, daß die Vorsprünge 5 außer in der mittigen Reihe 6a nicht auf Meridianen 14, sondern auf Parallelen, die den Pol 10 der Spreizpfanne 1 nicht schneiden, angeordnet sind. Dies wird insbesondere im rechten Bereich von Fig. 3 deutlich, wo der dem Pol 10 nächstliegende Vorsprung 5 ungeschnitten, der nächste teilweise geschnitten und der dem Äquator 2 nächstliegende Vorsprung 5 gänzlich geschnitten dargestellt ist. Wären die Vorsprünge 5 der Reihen 6b auf Meridianen 14 angeordnet, wären sie alle geschnitten.

Fig. 4 zeigt eine perspektivische Teildarstellung der erfindungsgemäß ausgestalteten Spreizpfanne 1 im Bereich IV in Fig. 2, wobei hier insbesondere die asymmetrische Form der Vorsprünge 5, welche in den benachbarten Reihen 6b achsenparallel zu den Vorsprüngen in der mittigen Reihe 6a ausgerichtet sind, deutlich wird.

Die Flanken 15 der Vorsprünge 5 der mittigen Reihe 6a stehen dabei senkrecht auf der Oberfläche der Spreizpfanne 1, während die Flanken 15a und 15b der Vorsprünge 5 der benachbarten Reihen 6b mit der Oberfläche der Spreizpfanne einen Winkel einschließen, der kleiner bzw. größer als 90° ist.

Die Erfindung ist nicht auf das dargestellte Ausführungsbeispiel beschränkt und beispielsweise auch für Spreizpfannen 1 mit unterschiedlichen Anzahlen von meridialen Schlitzen 3 bzw. Lappen 4 oder auch für von der Kugelform abweichende Formen der Spreizpfanne 1 geeignet.

## Patentansprüche

1. Spreizpfanne (1) für eine zementfrei verankerbare Gelenksendoprothese, wobei die Spreizpfanne (1) zumindest annähernd halbkugelförmig ausgebildet und mit mehreren, in einer Meridianrichtung verlaufenden Schlitzen (3) versehen ist, wodurch dazwischenliegende Lappen (4) ausgebildet sind, an denen Vorsprünge (5) ausgebildet sind, die mehrere von einem Äquator (2) der Spreizpfanne (1) ausgehende Reihen (6; 6a; 6b) bilden,
**dadurch gekennzeichnet,**
**daß** die Vorsprünge (5) so auf den Lappen (4) angeordnet sind, daß eine mittig auf dem Lappen angeordnete Reihe (6a) von Vorsprüngen (5) auf einem Meridian (14) der halbkugeligen Spreizpfanne (1) verläuft, während zumindest eine benachbarte Reihe (6b) von Vorsprüngen (5) auf einer Linie verläuft, die in jedem Punkt parallel zu dem Meridian (14) der mittig angeordneten Reihe (6a) von Vorsprüngen (5) und nicht durch einen Pol (10) der halbkugeligen Spreizpfanne (1) verläuft.

2. Spreizpfanne nach Anspruch 1,
**dadurch gekennzeichnet,**
**daß** die Anzahl der Reihen (6; 6a; 6b) von Vorsprüngen (5) pro Lappen (4) drei beträgt.

3. Spreizpfanne nach Anspruch 2,
**dadurch gekennzeichnet,**
**daß** die Abstände der Vorsprünge (5) der drei Reihen (6; 6a; 6b) auf jedem Lappen (4) gleich groß sind.

4. Spreizpfanne nach einem der Ansprüche 1 bis 3,
**dadurch gekennzeichnet,**
**daß** die Vorsprünge (5) auf konzentrischen Breitenkreisen angeordnet sind.

5. Spreizpfanne nach einem der Ansprüche 1 bis 4,
**dadurch gekennzeichnet,**
**daß** eine Höhe der Vorsprünge (5) über einer äußeren Oberfläche (9) der Spreizpfanne (1) mit zunehmender Entfernung der Vorsprünge (5) vom Äquator (2) der Spreizpfanne (1) abnimmt.

6. Spreizpfanne nach einem der Ansprüche 1 bis 5,
**dadurch gekennzeichnet,**
**daß** die Vorsprünge (5) quaderförmig mit einem aufgesetzten dreikantigen Prisma ausgebildet sind.

7. Spreizpfanne nach Anspruch 6,
**dadurch gekennzeichnet,**
**daß** die Vorsprünge (5) der mittigen Reihe (6a) symmetrisch geformt sind.

8. Spreizpfanne nach einem der Ansprüche 6 oder 7,
**dadurch gekennzeichnet,**
**daß** die Vorsprünge (5) der der mittigen Reihe (6a) benachbarten Reihen (6b) asymmetrisch geformt sind.

9. Spreizpfanne nach einem der Ansprüche 6 bis 8,
**dadurch gekennzeichnet,**
**daß** die Vorsprünge (5) auf den Lappen (4) so angeordnet sind, daß die auf den Lappen (4) mittig angeordnete Reihe (6a) von Vorsprüngen (5) senkrecht auf der Oberfläche (9) der Spreizpfanne (1) steht und Flanken (15; 15a; 15b) des quaderförmigen Teils der Vorsprünge (5) mit dieser Oberfläche (9) jeweils einen Winkel von ca. 90° einschließen.

10. Spreizpfanne nach einem der Ansprüche 6 bis 9,
**dadurch gekennzeichnet,**
**daß** die Vorsprünge (5) der der mittigen Reihe (6a) benachbarten Reihen (6b) parallel zu den Vorsprüngen (5) der mittigen Reihe (6a) stehen und die Flanken (15b) des quaderförmigen Teils der Vorsprünge (5) mit der Oberfläche (9) der Spreizpfanne (1) einen Winkel einschließen, der größer als 90° ist.

11. Spreizpfanne nach einem der Ansprüche 1 bis 10,
**dadurch gekennzeichnet,**
**daß** die Spreizpfanne (1) an ihrem Äquator (2) eine nach radial innen gerichtete Abschrägung (7) der Lappen (4) aufweist.

12. Spreizpfanne nach einem der Ansprüche 1 bis 11,
**dadurch gekennzeichnet,**
**daß** die Lappen (4) im Bereich der äquatorialen Abschrägung (7) jeweils eine Nut (8) aufweisen.

## Claims

1. Expandable socket (1) for a joint endoprosthesis able to be cementlessly anchored in place, the expandable socket (1) being at least approximately hemispherical in shape and being provided with a plurality of slots (3) extending in a meridional direction, thereby forming intervening tongues (4) on which are formed projections (5) which form a plurality of rows (6; 6a, 6b) extending from an equator (2) of the expandable socket (1), **characterised in that** the projections (5) are arranged on the tongues (4) in such a manner that a row (6a) of projections (5) arranged in the centre of the tongue extends on a meridian (14) of the hemispherical expandable socket (1) while at least one adjoining row (6b) of projections (5) extends in a line that extends in parallel at all points to the meridian (14) of the row (6a) of projections (5) arranged in the centre and not through a pole (10) of the hemispherical expandable socket (1).

2. Expandable socket (1) according to claim 1, **characterised in that** the number of rows (6 ; 6a, 6b) of projections (5) is three per tongue.

3. Expandable socket (1) according to claim 2, **characterised in that** the spacings of the projections (5) in the three rows (6; 6a, 6b) on each tongue (4) are the same.

4. Expandable socket (1) according to one of claims 1 to 3, **characterised in that** the projections (5) are arranged on concentric parallels of latitude.

5. Expandable socket (1) according one of claims 1 to 4, **characterised in that** a height of the projections (5) above an outer surface (9) of the expandable socket (1) becomes smaller as the distance of the projections (5) from the equator (2) of the expandable socket (1) becomes larger.

6. Expandable socket (1) according to one of claims 1 to 5, **characterised in that** the projections (5) are in the form of a parallelepiped surmounted by a three-edged prism.

7. Expandable socket (1) according to claim 6, **characterised in that** the projections (5) in the centre row (6a) are symmetrical in shape.

8. Expandable socket (1) according to either of claims 6 and 7, **characterised in that** the projections (5) in the rows (6b) adjoining the centre row (6a) are asymmetrical in shape.

9. Expandable socket (1) according to one of claims 6 to 8, **characterised in that** the projections (5) are so arranged on the tongues (4) that the row (6a) of projections (5) arranged centrally on the tongues (4) stands perpendicularly on the surface (9) of the expandable socket (1) and flanks (15; 15a, 15b) of the parallelepiped parts of the projections (5) each made an angle of approximately 90° with the said surface (9).

10. Expandable socket (1) according to one of claims 6 to 9, **characterised in that** the projections (5) in the rows (6b) adjoining the centre row (6a) are parallel to the projections (5) in the centre row (6a) and the flanks (15b) of the parallelepiped parts of the projections (5) make an angle of more than 90° with the surface (9) of the expandable socket (1).

11. Expandable socket (1) according to one of claims 1 to 10, **characterised in that** at its equator (2) the expandable socket (1) has a radially inwardly inclined bevel (7) on the tongues (4).

12. Expandable socket (1) according to one of claims 1 to 11, **characterised in that** the tongues (4) each have a grooves (8) in the region of the equatorial bevel (7).

## Revendications

1. Cotyle extensible (1) pour une endoprothèse d'articulation ancrable non cimentée, le cotyle extensible (1) étant configuré sous une forme au moins approximativement hémisphérique et comportant plusieurs échancrures (3) allant dans le sens méridien, ce par quoi des languettes (4) Intermédiaires sont formées, sur lesquelles des parties en saillie (5) sont constituées, qui forment plusieurs rangées (6, 6a, 6b) partant d'un équateur (2) du cotyle extensible (1),
**caractérisé en ce que**
les parties en saillie (5) sont disposées sur les languettes (4) de telle sorte qu'une rangée (6a) de parties en saillie (5) disposée de manière centrale sur les languettes progresse sur un méridien (14) du cotyle extensible (1) hémisphérique, tandis qu'au moins une rangée voisine (6b) de parties en saillie (5) progresse sur une ligne, qui progresse en tout point parallèlement au méridien (14) de la rangée de parties en saillie (5) disposée au centre (6a) et qui ne passe pas par un pôle (10) du cotyle extensible hémisphérique (1).

2. Cotyle extensible selon la revendication 1,
**caractérisé en ce que**
le nombre des rangées (6, 6a, 6b) des parties en saillie (5) par languette (4) est de trois.

3. Cotyle extensible selon la revendication 2,
**caractérisé en ce que**
les distances entre les parties en saillie (5) des trois rangées (6, 6a, 6b) sont de grandeur égale sur chaque languette (4).

4. Cotyle extensible selon l'une des revendications 1 à 3,
**caractérisé en ce que**
les parties en saillie (5) sont disposées sur des cercles de diamètres concentriques.

5. Cotyle extensible selon l'une des revendications 1 à 4,
**caractérisé en ce**
**qu'**une hauteur des parties en saillie (5) décroît au dessus d'une surface extérieure (9) du cotyle extensible (1) au fur et à mesure que les parties en saillie s'éloignent de l'équateur (2) du cotyle extensible (1).

6. Cotyle extensible selon l'une des revendications 1 à 5,
**caractérisé en ce que**
les parties en saillie (5) sont construites en forme de parallélépipède comportant un prisme triangulaire posé sur le dessus.

7. Cotyle extensible selon la revendication 6.
**caractérisé en ce que**
les parties en saillie (5) de la rangée centrale (6a) sont façonnées de manière symétrique.

8. Cotyle extensible selon l'une des revendications 6 ou 7,
**caractérisé en ce que**
les parties en saillie (5) des rangées (6b) voisines de la rangée centrale (6a) sont façonnées de manière asymétrique.

9. Cotyle extensible selon l'une des revendications 6 à 8,
**caractérisé en ce que**
les parties en saillie (5) sont disposées sur les languettes (4) de telle manière que la rangée (6a) de parties en saillie (5) disposée au milieu sur les languettes (4) est perpendiculaire à la surface (9) du cotyle extensible (1) et que les flancs (15, 15a, 15b) de la partie parallélépipédique des parties en saillie (5) incluent avec cette surface (9) respectivement un angle d'env. 90°.

10. Cotyle extensible selon l'une des revendications 6 à 9,
**caractérisé en ce que**
les parties en saillie (5) des rangées (6b) voisines de la rangée centrale (6a) sont parallèles aux parties en saillie (5) de la rangée centrale (6a) et les flancs de la partie parallélépipédique des parties en saillie (5) incluent un angle supérieur à 90° avec la surface (9) du cotyle extensible (1).

11. Cotyle extensible selon l'une des revendications 1 à 10,
**caractérisé en ce que**
le cotyle extensible (1) présente à son équateur (2) un biseau (7) des languettes (4) orienté vers l'intérieur en radial (7).

12. Cotyle extensible selon l'une des revendications 1 à 11,
**caractérisé en ce que**
les languettes (4) présentent dans la zone du biseau équatorial (7) à chaque fois une encoche (8).
